(19)

Europäisches
Patentamt
European
Patent Office
Office européen
des brevets

(11) **EP 4 436 473 B1**

(12) **EUROPEAN PATENT SPECIFICATION**

(45) Date of publication and mention
of the grant of the patent:
**18.06.2025 Bulletin 2025/25**

(21) Application number: **22821466.4**

(22) Date of filing: **22.11.2022**

(51) International Patent Classification (IPC):
**G06T 7/60** (2017.01)          **A61B 5/107** (2006.01)
**G06N 3/04** (2023.01)

(52) Cooperative Patent Classification (CPC):
**A61B 5/1075; G06T 7/60;** G06N 3/045;
G06T 2207/20081; G06T 2207/20084;
G06T 2207/20164; G06T 2207/30088;
G06V 10/225; G06V 10/32; G06V 10/454;
G06V 10/82; G06V 20/647

(86) International application number:
**PCT/EP2022/082801**

(87) International publication number:
**WO 2023/094377 (01.06.2023 Gazette 2023/22)**

(54) **A METHOD AND SYSTEM FOR BODY PART MEASUREMENT FOR SKIN TREATMENT**

VERFAHREN UND VORRICHTUNG ZUR KÖRPERTEILMESSUNG ZUR HAUTBEHANDLUNG

PROCÉDÉ ET SYSTÈME DE MESURE DE PARTIE DU CORPS POUR LE TRAITEMENT DE LA PEAU

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **24.11.2021  IN 202141054331
07.12.2021  EP 21212692**

(43) Date of publication of application:
**02.10.2024  Bulletin 2024/40**

(73) Proprietor: **Koninklijke Philips N.V.
5656 AG Eindhoven (NL)**

(72) Inventors:
• **MAITY, Reevu**
  **5656 AG Eindhoven (NL)**
• **PATIL, Ravindra**
  **5656 AG Eindhoven (NL)**
• **GUTTA, Srinivas**
  **5656 AG Eindhoven (NL)**

(74) Representative: **Philips Intellectual Property &
Standards
High Tech Campus 52
5656 AG Eindhoven (NL)**

(56) References cited:
• **CHINO DANIEL Y T ET AL: "Segmenting skin
ulcers and measuring the wound area using
deep convolutional networks", COMPUTER
METHODS AND PROGRAMS IN BIOMEDICINE,
ELSEVIER, AMSTERDAM, NL, vol. 191, 7
February 2020 (2020-02-07), XP086171958, ISSN:
0169-2607, [retrieved on 20200207], DOI: 10.1016/
J.CMPB.2020.105376**
• **ASLAM MURTAZA ET AL: "Automatic
measurement of anthropometric dimensions
using frontal and lateral silhouettes", IET
COMPUTER VISION, THE INSTITUTION OF
ENGINEERING AND TECHNOLOGY, MICHAEL
FARADAY HOUSE, SIX HILLS WAY, STEVENAGE,
HERTS. SG1 2AY, UK, vol. 11, no. 6, September
2017 (2017-09-01), pages 434 - 447,
XP006063098, ISSN: 1751-9632, DOI: 10.1049/
IET-CVI.2016.0406**

EP 4 436 473 B1

**Description**

FIELD OF THE INVENTION

The present invention relates to a method for use in a skin treatment device, and in particular, a method performed by one or more computers using neural networks for outputting a measurement of a dimension of a body part in an input image, a computer program product and a system thereof. The present invention further relates to a method of training neural networks used to control an electronic device to output the measurement of a dimension of a body part in an input image, a computer program and a method and system thereof for training the neural networks.

BACKGROUND OF THE INVENTION

It is typical for skin treatment devices to make use of software applications (apps) which can be downloaded on user electronic devices such as smartphones or tablets. These electronic devices are coupled to the skin treatment device, by means of connections such as Wireless Fidelity (WiFi) or Bluetooth. The apps can be used for controlling an operation of the skin treatment device, displaying messages to the user and the like.

For control actions relating to skin treatment, the app may prompt the user to input a dimension such as length of a body part via a suitable user interface (e.g. touchscreen) of the electronic device. Conventionally, the user manually measures the length of the body part to be treated by the skin treatment device, e. g. using a standard measuring tape. The input length is used by the app to calculate treatment parameters which are customized to the user. For example, in a skin treatment such as an Intense Pulsed Light (IPL) device, the treatment parameters include a number of light flashes, the intensity of light, etc. required to treat the body part.

CHINO DANIEL Y T ET AL : 'Segmenting skin ulcers and measuring the wound area using deep convolutional networks', COMPUTER METHODS AND PROGRAMS IN BIOMEDICINE, ELSEVIER, AMSTERDAM, NL, vol. 191, 7 February 2020, discloses an automatic skin ulcer region assessment framework to accurately segment a wound in a patient's skin ulcer image and automatically estimate its area and size. The assessment framework uses an encoder/decoder deep neural network to perform segmentation of the wound in the image. The assessment framework also detects a measurements ruler/tape present in the image and automatically estimates the pixel density of the image. This allows an accurate measurement of the size of the wound.

ASLAM MURTAZA ET AL: 'Automatic measurement of anthropometric dimensions using frontal and lateral silhouettes', IET COMPUTER VISION, THE INSTITUTION OF ENGINEERING AND TECHNOLOGY, MICHAEL FARADAY HOUSE, SIX HILLS WAY, STEVENAGE, HERTS. SG1 2AY, UK, vol. 11, no. 6, (2017-09), pages 434-447 discloses an automatic method for determining anthropometric dimensions of the human body using two-dimensional images. The method uses an efficient algorithm for automatically determining the anthropometric dimensions using fiducial points that are detected from frontal and lateral views of body silhouettes. The accuracy of the detected fiducial points is improved by segmenting the human body silhouette in both views. A large number of primary anthropometric dimensions are obtained by calculating the difference between two relevant fiducial points. A large number of secondary dimensional ratios are obtained directly from the primary dimensions, and circumferential dimensions are estimated using an ellipoid model.

SUMMARY OF THE INVENTION

The present invention is defined by the appended claims.

It is an object of the present invention to provide an improved solution which can automate measurement of a dimension of the body part, making it more convenient to the user.

The user is also prone to making errors in measurement, resulting in an inaccurate calculation of treatment parameters. It is another object of the invention to provide a solution which can improve the accuracy of the measured body part.

In the field of computer vision, human body key point detection relates to detecting human body key points or fiducial points in an image. However, the method requires complex image processing routines, thus making it slow for real time applications.

It is yet another object of the present invention to provide an automated solution addressing the challenges associated with image processing methods such as key point detection. This is achieved by use of a hybrid method based on neural network(s) and key point detection.

Neural networks are machine learning models that employ one or more layers of nonlinear units to predict an output for a received input. Some neural networks are deep neural networks which include one or more hidden layers in addition to an output layer. The output of each hidden layer is used as input to the next layer in the network, i.e., the next hidden layer or the output layer. Each layer of the network generates an output from a received input in accordance with current values of a respective set of parameters.

It is yet another object of the present invention to provide a technique for training neural networks, which networks can be used to output a measurement of a dimension of a body part in an input image on an electronic device.

The details of one or more embodiments of the subject matter of this specification are set forth in the accompanying drawings and the description below.

Other features, aspects, and advantages of the subject matter will become apparent from the description, the drawings, and the claims. For example, the specification provides methods, systems, and apparatus, including computer programs encoded on computer storage media, for carrying out any of the techniques disclosed herein. In some embodiments, a computer program comprises instructions that when executed by computing apparatus causes it to perform any of the techniques disclosed herein.

In one aspect, a method performed by one or more computers and a system thereof for outputting a measurement of a dimension of a body part in an input image is provided. The method includes receiving the input image captured using an image capturing unit, wherein the input image includes the body part and a reference object of a predefined dimension, predicting by a segmentation deep neural network, S-DNN, a segmentation mask of the input image, the segmentation mask differentiating image pixels of the body part from image pixels of a background region in the input image, detecting the reference object in the input image and processing the detected reference object to predict a pixel dimension of the reference object by an object detection deep neural network, OD-DNN, obtaining pixel coordinates of a plurality of key points of the body part using the segmentation mask, modifying the obtained pixel coordinates based on the predicted pixel dimension of the reference object, and measuring the dimension of the body part based on the modified pixel coordinates and outputting the measured dimension of the body part.

The method and system provide an improved measurement solution which can automate measurement of a dimension of the body part of the user, making a device incorporating the solution more user-friendly. As a result of the features used therein, the solution also improves the accuracy of the measured body part. The method further reduces the complexity of image processing routines, and makes them more suited to real time applications, and therefore can be implemented on devices with relatively less processing capability.

In one aspect, a method of training a plurality of neural networks and a system thereof used to control an electronic device to output a measurement of a dimension of a body part in an input image is provided. The input image is captured by an image capturing unit of the electronic device. The plurality of neural networks includes an object detection deep neural network, OD-DNN, and a segmentation deep neural network, S-DNN. The method includes the steps of initializing neural network parameter values of the S-DNN and OD-DNN, obtaining a training data set including a plurality of images, each image including the body part and a reference object of a predefined dimension, annotating the reference object and the body part in the images of the training data set to generate an annotated training data set, dividing a part of the annotated training data set into a validation data set, inputting the annotated training data set to the OD-DNN such that the OD-DNN learns to detect the reference object in the input image and proces the detected reference object to predict a pixel dimension of the reference object, inputting the annotated training data set to the S-DNN such that the S-DNN learns to predict a segmentation mask of the input image, wherein the segmentation mask differentiates image pixels of the body part from image pixels of a background region in the input image, validating, using the validation data set, a pixel dimension of the reference object learnt by the OD-DNN and a segmentation mask learnt by the S-DNN, and updating the neural network parameter values of the S-DNN and OD-DNN based on the validation.

The method and system provide a training solution for real world applications including controlling an electronic device to output a measurement of a body part. The output may be displayed to a user of the electronic device. In some implementations, the training solution continuously interacts with measurement input which is captured by one or more physical image capturing units.

In one aspect, one or more computer program products which include instructions which, when the program is executed by one or more computers, cause the one or more computers to carry out steps of the above-mentioned methods, are provided.

As mentioned, other features, aspects, and advantages of the subject matter will become apparent from the description, the figures, and the claims.

BRIEF DESCRIPTION OF THE DRAWINGS

    FIG. 1 illustrates a method 100 for outputting a measurement of a dimension of a body part in an input image in accordance with one embodiment.
    FIG. 2 illustrates a segmentation mask in accordance with one embodiment.
    FIG. 3 illustrates a reference object in accordance with one embodiment.
    FIG. 4 illustrates an aspect of image pixel measurement in accordance with one embodiment.
    FIG. 5A illustrates an aspect of key point method in accordance with one embodiment.
    FIG. 5B illustrates another aspect of key point method in accordance with one embodiment.
    FIG. 6 illustrates a system 600 for outputting a measurement of a dimension of a body part in accordance with one embodiment.
    FIG. 7 illustrates a method 700 for training a neural network in accordance with one embodiment.
    FIG. 8 illustrates a training system 800 in accordance with one embodiment.

DETAILED DESCRIPTION OF THE EMBODIMENTS

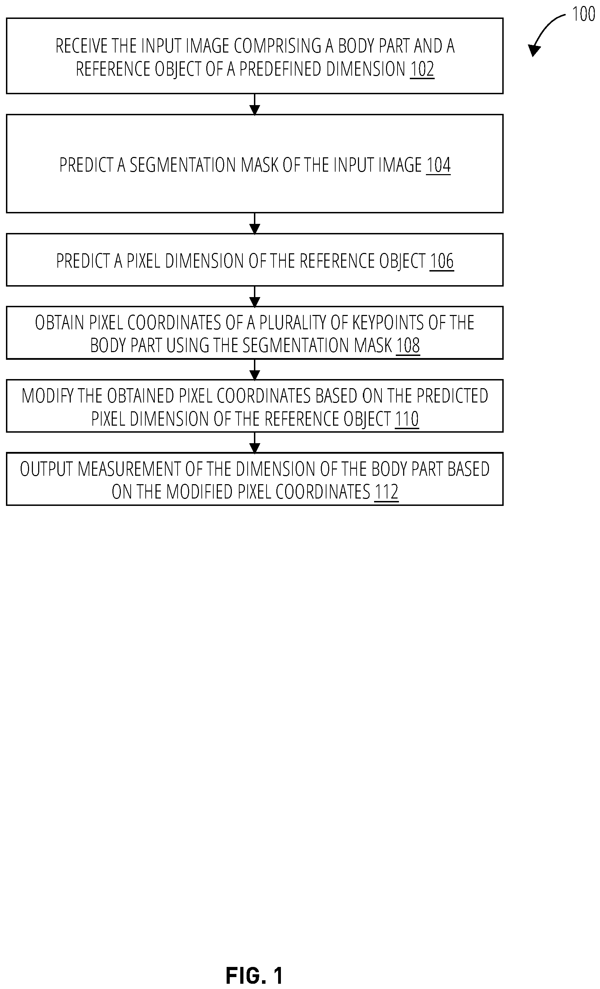

FIG. 1 is a flow chart of an example method 100 performed by one or more computers in a system for outputting a measurement of a dimension of a body part in an input image. An example of a dimension is length, but the present invention is not limited to measuring length. Other dimensions include but are not limited to breadth or height of the body part. For convenience, the method 100 will be described as being performed by a system of one or more computers located in one or more locations. For example, an image processing system with distributed architecture, appropriately programmed in accordance with this specification, can perform the method 100. Method 100 may also be performed by a single computer, for example, by a processor of a user electronic device/computer. The order of the steps of method 100 is merely exemplary, for example, the skilled person is aware that it is possible to interchange orders of at least some of the steps of the method. The term "user" is any user who uses the method described herein and/or the system and/or a computer program based on the method and need not be the same person as the subject on which the treatment is carried out. The subject may be a human or an animal.

In step 102, method 100 comprises receiving an input image. The input image comprises the body part which is intended to be treated, for example, a leg, an arm, or any other body part. It may be a two-dimensional (2D) image (e.g., represented as a 2D array of pixels) and may be acquired by an image scanner of any modality, for example, an image capturing unit of a user electronic device (smartphone camera).

The input image further comprises a reference object of a predefined dimension. It is difficult to obtain an accurate length of a region of interest (ROI) or desired object in a captured image. This is because, based on factors such as selected optical/digital zoom of the image capturing unit, image resolution, the angle of image capture, etc., the measurements of the ROI in an image may deviate from its actual values. Since all objects of an image share similar characteristics, such deviations may be compensated by referencing the ROI to another object in the same image, the other object having a known length.

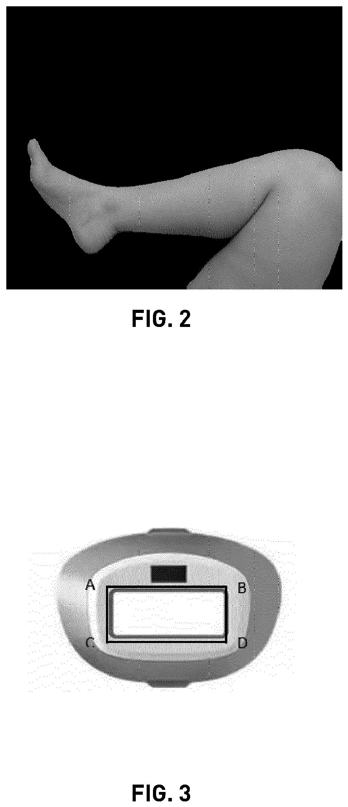

Skin treatment devices are often equipped with at least one attachment with a standard length. In a particular example, a treatment aperture of this attachment can be used as the reference object. An example of a reference object is shown in FIG. 2. In an IPL device (e.g. Philips Lumea), this could be the treatment aperture of a device attachment which is supplied with the IPL device. It has a known dimension of around 30 mm, specifically, 32.7 mm. It is understood that other dimensions are equally possible for the treatment aperture. In an example, the user is prompted by the app to capture an input image of the body part keeping the device attachment in the field of view of the camera.

In step 104, method 100 comprises predicting at least one segmentation mask of the input image. A segmentation deep neural network (S-DNN) is used to process the input image. In an example, a ResNet model is used. The S-DNN is configured to receive the input image and process the input image in accordance with current (optimised) values of S-DNN parameters to predict the segmentation mask. Step 104 may be performed at a single location by a single computer (for example, at the user electronic device) or at a different location by another computer or processor. In the latter example, the S-DNN may be located in a distributed server such as a cloud. Hence, the computing is performed at the server location. The user electronic device can be configured to transmit the acquired input image to the server by means of wireless communication. In this example, the user electronic device and the server form a plurality of computers in a wireless communication system. Since the image processing is carried out apart from the user (client) electronic device, less resources are needed by the device at the user location.

The S-DNN is a semantic classifier, which classifies pixels in the input image into a plurality of classes or types. In an example, a predicted segmentation mask comprises the classes ROI and background. The image pixels of the ROI in the segmentation mask relate to a first class and the image pixels of the background region in the segmentation mask relate to a second class. The second class relating to the background comprises those image pixels outside a boundary of the ROI or the body part, and can optionally be filtered from the segmentation mask (shown darkened in FIG.3). In another example, a plurality of segmentation masks are generated. A first segmentation mask relates to the first class, and a second segmentation mask relates to the second class. In other words, the segmentation mask differentiates image pixels of the ROI, the body part, from image pixels of a background region in the user image. An example of a segmentation mask showing the first/ROI class is shown in FIG. 3. The remaining steps of processing the image are carried on this predicted segmentation mask - a subset of the input image, making the process faster than while processing raw input image data.

In some implementations, the segmentation mask has a score, which represents a likelihood that the predicted mask is the most appropriate classification for the user. This score, amongst other parameters, may be used by the S-DNN for future predictions, i.e., for computing the segmentation mask of another input image of the same or similar body part at a later point of time.

As mentioned, the input image comprises the reference object of a predefined dimension/length/size, in order to obtain an accurate measurement of the ROI in the input image. In step 106, method 100 detects the reference object in the input image and processes the reference object in the input image to predict an image pixel dimension, e.g., a (total) pixel length of the reference object, in other words, the length of a row of pixels (number of pixels) spanning the length of the reference object in the input image. The prediction acts as a correction factor and is applied to pixel coordinates of the ROI (body part) determined via keypoint detection. This is detailed below.

An object detection deep neural network (OD-DNN) is used to detect and process the reference object in the input image. In an example, a You only look once (YOLO) object detection convolutional neural network (CNN) model is used. The OD-DNN is configured to receive and process the input image in accordance with current (optimised) values of OD-DNN parameters to predict the pixel length of the reference object. The OD-DNN may further be configured to process the input image in accordance with current (optimised) values of OD-DNN parameters to detect the ROI. Like step 104, step 106 may be performed at a single location by a single computer (for example, at the user electronic device) or at a different location by a distributed computer. In the latter example, the OD-DNN may be located in a distributed server such as a cloud. Hence, the processing or computing is performed at the server location. Similar to step 104, the user electronic device can be configured to transmit the acquired input image to the server by means of wireless communication, the electronic device and the server forming a wireless communication system. Since the image processing is carried out apart from the user (client) electronic device, less resources are needed by the device at the user location.

In some implementations, the OD-DNN is trained to output pixel coordinates of a bounding box which outlines the reference object. A bounding box shows the spatial location of an object and is presented by a set of coordinates that tends to bound the object by the smallest enclosing box. An example of a bounding box is shown in FIG. 2. While training the OD-DNN, the bounding box technique can be used to annotate or label the reference object in each image of a training image data set, so that the OD-DNN learns to obtain a pixel length from the predicted pixel coordinates of the bounding box in the input image with a degree of accuracy.

Like in step 104, the predicted pixel length of the reference object has a score, which represents a likelihood that the prediction is the most appropriate (linear) regression. This score, amongst other parameters, may be used by the OD-DNN for future predictions.

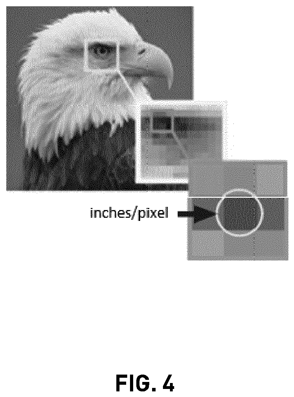

In some implementations, the OD-DNN calculates a single/per pixel length, denoted as a measurement per pixel (MPP), based on the predefined dimension and the predicted pixel dimension of the reference object. In the field of computer vision, MPP is understood to quantify the number of inches or centimeters of the environment shown in the iamge encoded or represented per pixel length or per pixel breadth of the image. An example is shown in FIG. 4. The task performed by the OD-DNN is then to determine how much real-world spatial length is encoded per pixel length or per pixel breadth of the image.

MPP is calculated as:

$$MPP = \frac{Ground\ truth\ length\ of\ object}{Pixel\ length\ of\ object}$$

In the above equation, the predefined dimension of the reference object is denoted by the term ground truth length, and the predicted pixel dimension is denoted as the pixel length of the object. This metric acts as a correction factor and is applied to pixel coordinates of the ROI (body part) determined via keypoint detection. Some other advantages of measuring (reference) objects in an image with the MPP are that the metric is independent of the distance between an image capturing unit (camera) and the object, that it can be employed for any object of known dimensions in the image, is independent of the pixel density of the image and of camera calibration parameters such as skew coefficient and optical center.

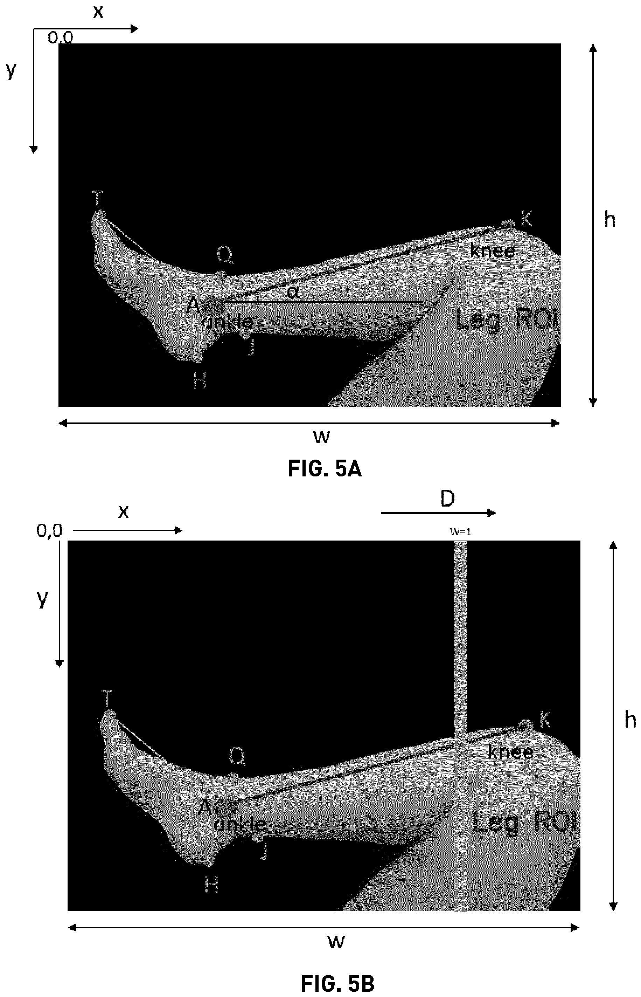

In step 108, method 100 obtains pixel coordinates of a plurality of key points of the body part from the segmentation mask obtained in step 104. This is shown in FIGs. 5A and 5B. The key point detection algorithm used in the invention is especially chosen to improve the resource usage of the method and/or the user electronic device, in other words, its internal functioning. Built on a non-AI based model, it has the advantage that it does not require prior training to perform a task. In contrast, an AI model typically requires large amounts of training data to learn the underlying patterns or features in the data. As such, it is suited to be implemented on the user electronic device. As mentioned, the algorithm processes a sub-set of the image data, making it faster to implement on a user device. In some embodiments, step 108 is performed by a remote/distributed computer in the system.

In an example, the key points of body part "lower leg" are chosen as the ankle and knee joints. An example of how these key points are detected is as follows. In FIG. 5A, the segmentation mask shows details of an image of the lower leg at a certain orientation. The height of the mask is h pixels, and the width is w pixels. A set of coordinate axes x (horizontal) and y (vertical) is defined with respect to the segmentation mask, with an arbitrary origin defined as (0,0). In FIGs. 5A and 5B, the origin is located at the top-left corner. It is preferred that the lower leg is aligned at a non-zero angle $\alpha$ with respect to the horizontal axis of the mask. The recommended lower leg orientation allows to determine points of the desired key points using elementary calculus in a straightforward manner. The ankle and knee joints are identified as follows. Given the orientation of the leg, the leg outline pixel on the mask associated with a y-local minimum is assigned as the knee joint K. This is as shown in the right half portion of FIG. 5A. In other words, the pixel with the minimum y-coordinate is treated as the knee joint coordinate. To determine the ankle joint A, the method determines a set of four points T, H, Q and J in the lower leg, and the ankle joint co-ordinate A is determined by the point of intersection between the line segments TJ and HQ. A second local minimum y-coordinate point in the mask is determined as a toe pixel T. A local maximum y-coordinate point is determined as a heel pixel H. A local maximum point near an upper intersection of the foot and leg in the mask is selected as point Q, and point J is empirically defined as x(H) + 100 units where x(H) is the x-coordinate of the heel H.

An exemplary implementation to calculate the local minima/maxima is shown in FIG. 5B. A vertical slider of pixel width 1 can be scanned across the segmentation mask in a given direction. In FIG. 5B, for identifying the knee joint K, the vertical slider scans the mask in a given direction D, for example, left to right. The set of coordinate axes is defined, as mentioned above. The vertical slider identifies all ROI pixels on the mask outline (boundary) along the scanning direction and checks for the height of the pixel along said outline. The pixel with the lowest height with respect to the assigned y=0 is chosen as the knee joint pixel y coordinate. The corresponding x coordinate is also identified. The implementation can be repeated for detection of each key point. It is clear to the skilled person that any identification means such as a cursor can be used instead of the vertical slider.

In some embodiments, the method calculates the distance between the obtained key points.

In step 110, method 100 modifies the obtained pixel coordinates of the key points or the distance between the key points based on the predicted pixel dimension of the reference object. In some implementations, the method corrects the obtained coordinates using the pixel size derived from the measurement per pixel calculation in accordance with the equation in paragraph [38], hence, actual lower leg length = MPP * distance between the key points (pixel length of the ROI).

Such correction, based on reference to an object in the input image with known dimensions, accounts for ROI size variations due to factors such as selected optical/digital zoom of the image capturing unit, image resolution, the angle of image capture, etc.

In step 112, method 100 outputs the measurement of the length of the body part based on the modified pixel coordinates. The measurement may be displayed by the user electronic device to the user by means of its display screen. The measured value is further transmitted to the skin treatment device for calculation of treatment parameters by the user electronic device. In some embodiments, the measured value can be directly transmitted to the skin treatment device, i.e., without further display to the user.

The method may use at least one of the input image, segmentation mask, the predicted pixel dimension, and the output measurement of the dimension of the body part for future predictions of the segmentation mask, the pixel dimension, and the output measurement of the dimension of the body part.

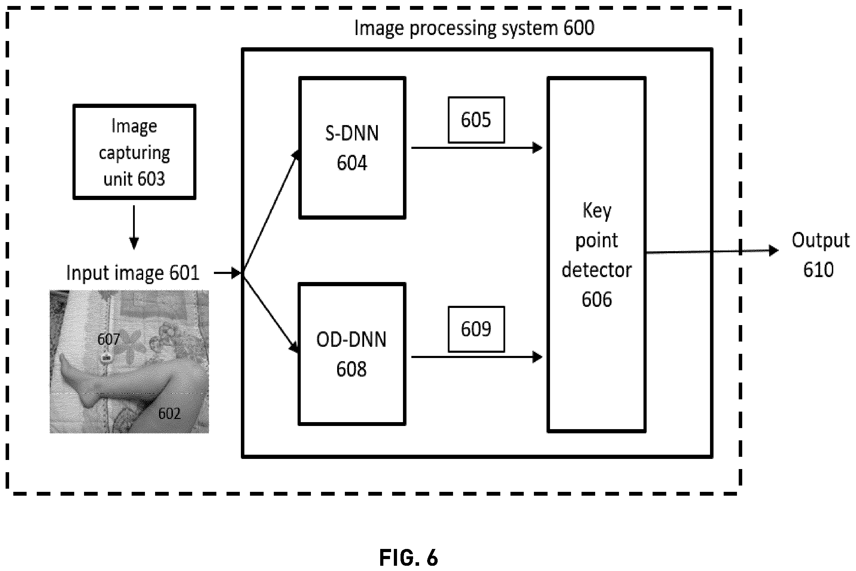

FIG. 6 illustrates an example image processing system 600 according to an exemplary embodiment of the present invention. It is an example of a system implemented as computer programs on one or more computers in one or more locations in which the systems, components, and techniques described herein are implemented.

The image processing system 600 is configured to receive and process an input image 601 depicting or including a body part to be treated. The body part is considered the ROI 602 in the input image. Input image 601 is acquired using an image capturing unit 603 which may form part of system 600, or be external to it. As mentioned, the image capturing unit 603 may be part of a user electronic device, and the body part may be that of the user or another subject.

The system 600 processes the input image 601 to predict a segmentation mask of the input image. In doing so, it provides the input image as an input to an S-DNN 604 (as mentioned above). The S-DNN is configured to receive the input image 601 and process it in accordance with current values of S-DNN parameters to generate or predict a segmentation mask 605 (based on a hypothesis that approximates a function between an input to the DNN and an expected output). The segmentation mask characterizes a segmentation of the input image into at least one class, the class comprising image pixels of the ROI, the body part.

The system 600 may store the generated segmentation mask in a data store (e.g., a logical data storage area or a physical data storage device, not shown) wirelessly coupled to the system 600. A user of the system 600 (e.g., a user operating a device at home) may subsequently retrieve the segmentation mask from the data store. In some cases, the system 600 may directly present the generated segmentation mask on a display device (e.g., a computer screen) visible to a user of the system 600, e.g., using the display of the user electronic device.

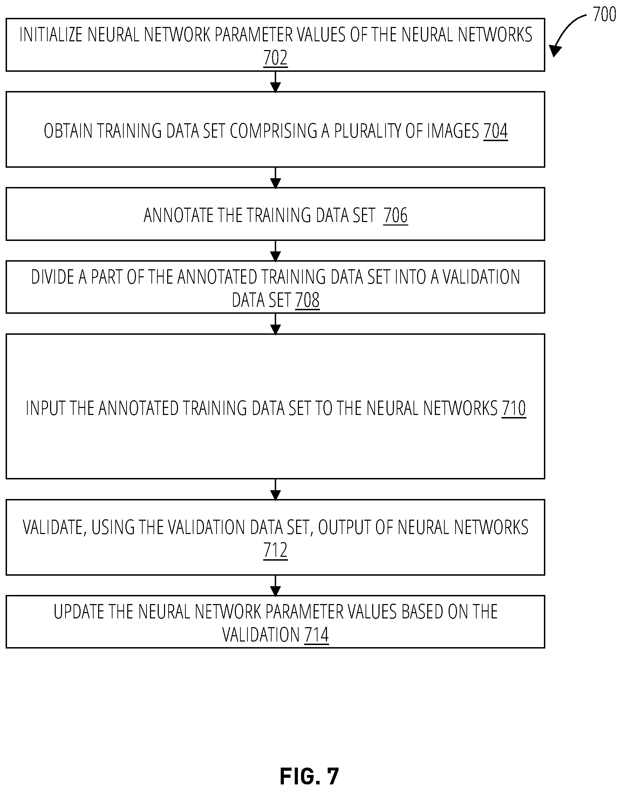

The S-DNN is trained with differently initialized parameter values, on different training images, or both. An example process for training the S-DNN is described with reference to FIG. 7. In some implementations, the S-DNN is pre-trained to predict segmentation masks of arbitrary images. The pre-trained models are further trained using customized training data images. The advantage of using pre-trained models are that the calculations converge faster, i.e., the optimized neural network parameters are obtained faster. In an example, the S-DNN is a semantic classifier. Some model architectures which can perform semantic segmentation include fully convolutional network (FCN), U-Net, Mask RCNN, etc. In an example, the S-DNN may be implemented using DeepLabv3 PyTorch model with an FCN-ResNet101 backbone.

To generate the output measurement 610 of the body part, the system 600 further provides the segmentation mask 605 to the key point detector 606. The algorithm or model of the key point detector 606 may be embedded in a user device which is comprised internal or external to system 600, or at a server location together with S-DNN and/or other neural networks (the latter shown in FIG. 6). The implementation of the key point algorithm is as mentioned above. The key point detection algorithm can highly improve latency of predictions since the algorithm does not rely on resource-intensive deep neural network architectures for identifying the image pixels corresponding to the key points in the ROI. The output measurement of the body part comprises a dimension of the body part determined by locating key points among the ROI/body part image pixels in the segmentation mask. Optionally, system 600 outputs the measurement to a user device.

In some implementations, the system 600 processes the input image 601 further to modify the obtained dimension. In this case, input image 601 additionally includes a reference object 607. The system 600 provides the input image 601 as an input to an OD-DNN 608 (specifics of OD-DNN as mentioned above). The OD-DNN processes the reference object 607 to predict a pixel dimension of the reference object 609. In some implementations, the system 600 further calculates a pixel size based on the predicted pixel dimension of the reference object and a known dimension of the reference object 607. System 600 corrects the obtained dimension of the body part 610 based on the predicted pixel dimension of the reference object or the derived pixel size/metric MPP. The key point detector 606 may apply this correction to the obtained pixel coordinates of the key points or the dimension of the body part calculated based on these pixel coordinates in its output 610.

The OD-DNN is trained with differently initialized parameter values, on different training images, or both. An example process for training the OD-DNN is described with reference to FIG. 7. In some implementations, the OD-DNN is pre-trained to predict pixel dimensions of arbitrary images. The pre-trained OD-DNN is further trained using customized training data images. In an example, the OD-DNN may be implemented using a Yolo AI model. Depending on the extent of accuracy desired in prediction of dimension of the body part, a heavyweight or lightweight (terms well-known in art) YOLO model may be used. Other object detection neural network architectures include fast R-CNN, single shot detector (SSD) etc.

Both S-DNN and OD-DNN may be trained using separate supervised learning procedures. The neural networks may also be trained by the same supervised training procedure, using the same training data set. The procedures may involve using manually labelled training images. An example of the latter approach is illustrated in FIG. 7.

FIG. 7 is a flow chart of an exemplary method 700 for training an S-DNN and an OD-DNN according to an embodiment of the invention. The method 700 will be described as being performed by a system of one or more computers located in one or more locations. While it is possible to implement the method 700 using the system 600, it is advantageous to train the neural networks using a separate procedure, prior to implementing them in the method of FIG. 100. The trained OD-DNN and S-DNN can be used to predict the segmentation mask and the pixel dimension of the body part in the input image in method 100. As mentioned, the input image may be captured by an image capturing unit of the electronic device. The electronic device may be one of the one or more computers in the system. Hence, the training procedure of FIG. 7 can be used to control an electronic device to obtain and/or output a measurement of a dimension of a body part in an input image.

In step 702, the method comprises initializing the neural network parameter values of the S-DNN and OD-DNN. In some implementations, the method may initialize the S-DNN and OD-DNN neural network parameter values based on trained parameter values of another neural network with the same architecture as the S-DNN and OD-DNN, respectively, and which have been trained to perform similar predictions. For example, as mentioned above, S-DNN and OD-DNN may build on pre-trained models, i. e., the OD-DNN and S-DNN are prior trained to detect objects in an arbitrary image and semantically segment an arbitrary image into at least one segment mask of a specific image class, respectively. In this case the initialization can be based on trained parameter values of the pre-trained S-DNN and OD-DNN.

In some implementations, the score of the predictions in method 100 by system 600 is considered at step 702 during initialization of the parameter values, so that the training of the neural networks can be adjusted every time method 100 is performed. As a result, over time, the system 600 learns to obtain and/or output a measurement of a dimension of a body part in an input image to a user of an electronic device with improved accuracy.

In step 704, method 700 obtains a training examples or a training data set comprising a plurality of images, each image including the body part and a reference object of a predefined dimension. Each image in the training data set is further annotated by a human expert, in an automated or semi-automated manner. Annotation refers to a process by which a person, manually or using a computer, assigns a type to an object in the image for purposes of training a neural network.

In some implementations, method 700 obtains the training data set periodically from one or more computers in the system. For example, each time method 100 is performed by system 600, the input image may be stored in the system for later training purposes. In this manner, the S-DNN and OD-DNN can be iteratively trained.

In step 706, the method comprises annotating the reference object and the body part in the obtained training data set, to generate an annotated training data set.

In an example, in each image of the training data set, the image pixels of a particular class or particular classes in the training data set are annotated to generate an expected segmentation mask output. The annotated training data set is input to the S-DNN. Each example in the training data set input to the S-DNN has (i) a training image (input) and (ii) an annotated training segmentation mask showing image pixels of the body part class (expected output) in the training image. The S-DNN learns to map each input to each output using a possible hypothesis function.

In a training data set to the OD-DNN, the method comprises annotating the reference object in each image using annotation techniques such as by using the bounding box. As shown in FIG.2, the treatment aperture of a device attachment can be used as the reference object, therefore, the aperture can be annotated with a bounding box ABCD in each image of the training data set. Each example in the training data set to the OD-DNN has (i) a training image (input) and (ii) an annotated reference object (expected output) in the training image.

It is advantageous to modify the training data set to increase or augment the training set size, and hence the accuracy of model predictions. In some implementations, the method comprises generating different versions of each image in the training data set, using image augmentation techniques such as image translation and image flipping. Image augmentation also addresses the issue of overfitting of S-DNN and OD-DNN. With image augmentation, it is possible to obtain heterogeneous data that helps the models learn different patterns, hence avoiding overfitting.

In step 708, the method comprises dividing or splitting a part of the annotated training data set into a validation data set. A part of the annotated training data set is chosen as the validation data set. It is thus ensured that the training and validation sets are obtained using the same distribution of data. This subset is used to evaluate the performance of the S-DNN and OD-DNN in their predictions.

In step 710, the method comprises inputting the annotated training data set to the OD-DNN and the S-DNN such that the OD-DNN and the S-DNN learn to detect the reference object in the input image and process the detected reference object to predict a pixel dimension of the reference object, and to predict a segmentation mask of each image, respectively. The segmentation mask differentiates the image pixels of the ROI from the background in the image.

Step 712 comprises validating the segmentation mask and the pixel dimension of the reference object learnt by the trained S-DNN and OD-DNN, respectively, using the validation data set.

In an example, a trained YOLO OD-DNN achieves a mAP score of 0.70 on the validation data set. For each image in the validation set, the YOLO OD-DNN detects the reference object and predicts pixel co-ordinates A, B, C, D of a bounding box bounding the reference object. A trained DeepLab S-DNN achieves a mIOU score of 0.75 on the validation data set. For each image in the validation set, the DeepLab S-DNN predicts the segmentation mask showing image pixels of the ROI/body part.

Step 714 comprises updating the neural network parameter values of the S-DNN and OD-DNN based on the validation. The updated parameter values can further be used for testing the OD-DNN and S-DNN using a test data set comprising at least one image showing the body part and the reference object.

In some implementations, the pixel dimension of the reference object learnt by the OD-DNN is a single pixel size, preferably a measurement per pixel, calculated based on the predefined dimension and the predicted pixel dimension.

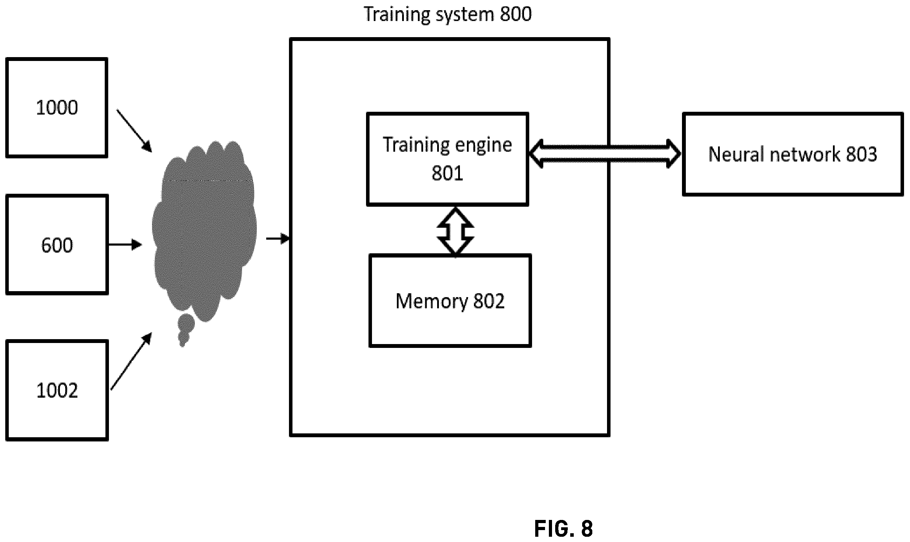

FIG.8 shows an example of a system 800 for training neural networks 803 according to the method of FIG. 7. The neural network training system 800 is an example of a system implemented on one or more computers in one or more locations, for example, as computer programs. System 800 may be implemented on system 600 (in other words, they be one and the same system), or as a separate system 800 wirelessly coupled to system 600. There may be additional systems 1000, 1002, ... coupled to system 800. Systems 1000, 1002 may be other training systems or image processing systems which may be configured to perform the method 100 or database management systems whose data may be used by system 800 to train neural networks 803.

System 800 includes a training engine 801 which has access to a memory 802 shared by the one or more computers in the system for training neural networks S-DNN and OD-DNN. In some implementations, training engine 801 is a distributed processor.

Training engine 801 may be configured to initialize the neural network parameters of the S-DNN and the OD-DNN. It retrieves the parameters from memory 801. Memory 801 may store trained parameter values of other neural networks with the same architecture as the S-DNN and OD-DNN, respectively, and which have been trained to perform similar predictions. If the S-DNN and OD-DNN build on pre-trained models, memory 801 may store trained parameter values of the pre-trained S-DNN and OD-DNN, respectively.

Training engine 801 may be further configured to obtain the training data set stored in memory 802, including the annotated training set. In some implementations, the training set stored in memory 802 is periodically updated as mentioned above. The continually updated training set can be retrieved by the training engine 801. Training engine 801 further obtains the pixel dimension of the reference object and the segmentation mask of each image in the training set predicted by the OD-DNN and S-DNN, respectively, validates the prediction (checks whether training criteria are satisfied, in some implementations, iteratively) and updates the neural network parameters of the S-DNN and the OD-DNN based thereupon, to reach optimized neural network parameters of the S-DNN and the OD-DNN. The trained neural networks S-DNN and the OD-DNN can be used to control an electronic device to obtain and/or output a measurement of a dimension of a body part in an input image. The optimized neural network parameters are used in a next initialization by training engine 801.

This specification uses the term "configured" in connection with systems and computer program components. For a system of one or more computers to be configured to perform particular operations or actions means that the system has installed on it software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non-transitory storage medium for execution by, or to control the operation of, data processing apparatus. The computer storage medium can be a machine-readable storage device, a machine-readable storage substrate, a random or serial access memory device, or a combination of one or more of them. Alternatively or in addition, the program instructions can be encoded on an artificially-generated propagated signal, e.g., a machine generated electrical, optical, or electromagnetic signal, that is generated to encode information for transmission to suitable receiver apparatus for execution by a data processing apparatus.

The terms "computer", "processor", "data processing apparatus", "electronic device" etc. refer to data processing hardware and encompasses all kinds of one or more apparatus, devices, and machines for processing data. The apparatus can also be, or further include, special purpose logic circuitry, e.g., an FPGA (field programmable gate array) or an ASIC (application-specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub-programs, or portions of code. A computer program can be deployed to be executed on one computer or on multiple computers that are located at one site or distributed across multiple sites and interconnected by a data communication network.

In this specification the term "engine" is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read-only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks.

To provide for interaction with a user, embodiments of the subject matter described in this specification can be implemented on a computer or electronic device having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Data processing apparatus or computers for implementing machine learning models can also include, for example, special-purpose hardware accelerator units for processing common and compute-intensive parts of machine learning training or production, i.e., inference, workloads.

Machine learning models can be implemented and deployed using a machine learning framework, e.g., a TensorFlow framework, a Microsoft Cognitive Toolkit framework, an Apache Singa framework, or an Apache MXNet framework.

Embodiments of the subject matter described in this specification can be implemented in a computing system that includes a back-end component, e.g., as a data server, or that includes a middleware component, e.g., an application server, or that includes a front-end component, e.g., a client computer having a graphical user interface, a web browser, or an app through which a user can interact with an implementation of the subject matter described in this specification, or any combination of one or more such back-end, middleware, or front-end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have thus been described for illustrative purposes while the invention is defined by the appended claims.

**Claims**

1. A method (100) performed by one or more computers for outputting a measurement (610) of a dimension of a body part (602) in an input image (601), the method comprising:

   receiving (102) the input image captured using an image capturing unit (603), the input image comprising the body part and a reference object (607) of a predefined dimension;
   predicting (104) by a segmentation deep neural network, S-DNN (604), a segmentation mask (605) of the input image, the segmentation mask differentiating image pixels of the body part from image pixels of a background region in the input image;
   detecting the reference object in the input image and processing the detected reference object to predict (106) a pixel dimension (609) of the reference object by an object detection deep neural network, OD-DNN (608);
   obtaining (108) pixel coordinates of a plurality of key points (A, H, J, K, Q, T) of the body part using the segmentation mask;
   modifying (110) the obtained pixel coordinates based on the predicted pixel dimension of the reference object; and
   measuring the dimension of the body part based on the modified pixel coordinates and outputting (112) the measured dimension of the body part.

2. The method (100) according to claim 1, wherein the S-DNN (604) is a semantic S-DNN such that the image pixels of the body part (602) in the segmentation mask (605) relate to a first class and the image pixels of the background region in the segmentation mask relate to a second class.

3. The method (100) according to claim 1 or claim 2, wherein the OD-DNN (608) is configured to calculate a measurement per pixel, MPP, corresponding to a dimension of the environment shown in the input image (601) represented by a single pixel, based on the predefined dimension and the predicted pixel dimension (609) of the reference object (607).

4. The method (100) according to any of the above claims, further comprising:
using at least one of the input image (601), the segmentation mask (605), the predicted pixel dimension (609) of the reference object (607) and the output measurement (610) of the dimension of the body part (602) for a future prediction of the segmentation mask and/or the pixel dimension of the reference object.

5. The method (100) of any of the preceding claims, wherein obtaining (108) pixel coordinates of a plurality of key points (A, H, J, K, Q, T) of the body part (602) using the segmentation mask (605) comprises:

    defining a plurality of coordinate axes (x, y) on the segmentation mask;
    identifying image pixels having local maxima and/or local minima coordinates along at least one chosen coordinate axis amongst the plurality of coordinate axes, of specific regions of the body part on the segmentation mask and based on the identified pixels of the specific regions, obtaining the pixel coordinates of the plurality of key points of the body part.

6. A computer program product comprising instructions which, when the program is executed by one or more computers, cause the one or more computers to carry out steps (102, 104, 106, 108, 110, 112) of the method (100) according to any of the preceding claims.

7. A system (600) comprising one or more computers and one or more storage devices storing instructions that when executed by the one or more computers, cause the one or more computers to perform the method (100) of any of claim 1 to claim 5.

8. A computer-implemented method (700) of training a plurality of neural networks (604, 608) used to control an electronic device (600) to output a measurement (610) of a dimension of a body part (602) in an input image (601), the input image captured by an image capturing unit (603) of the electronic device, the plurality of neural networks comprising an object detection deep neural network, OD-DNN (608) and a segmentation deep neural network, S-DNN (604), the method comprising the steps:

initializing (702) neural network parameter values of the S-DNN and OD-DNN;
obtaining (704) a training data set comprising a plurality of images, each image including the body part and a reference object (607) of a predefined dimension;
annotating (706) the reference object and the body part in the images of the training data set to generate an annotated training data set;
dividing (708) a part of the annotated training data set into a validation data set;
inputting (710) the annotated training data set to the OD-DNN such that the OD-DNN learns to detect the reference object in the input image and proces the detected reference object to predict a pixel dimension (609) of the reference object;
inputting (710) the annotated training data set to the S-DNN such that the S-DNN learns to predict a segmentation mask (605) of the input image, wherein the segmentation mask differentiates image pixels of the body part from image pixels of a background region in the input image;
validating (712), using the validation data set, a pixel dimension of the reference object learnt by the OD-DNN and a segmentation mask learnt by the S-DNN, and
updating (714) the neural network parameter values of the S-DNN and OD-DNN based on the validation.

9. The method (700) of claim 8, wherein the OD-DNN (608) and S-DNN (604) are prior trained to detect objects in an arbitrary image and semantically segment an arbitrary image into at least one segment mask of a specific image class, respectively.

10. The method (700) of claim 8 or claim 9, wherein annotating (706) the reference object (607) and the body part (602) in the obtained training data set comprises, respectively:

    annotating the reference object comprised in each image of the training data set using a bounding box;
    annotating image pixels of the body part in each image of the training data set, wherein the body part relates to a first class.

11. The method (700) according to any of the preceding claim 8 to claim 10, wherein the OD-DNN (608) is further learnt to calculate a measurement per pixel (MPP), corresponding to a dimension of the environment shown in the input image (601) represented by a single pixel, based on the predefined dimension and the predicted pixel dimension (609) of the reference object (607).

**12.** The method (700) according to any of the preceding claim 8 to claim 11, further comprising periodically obtaining the training data set comprising the plurality of images for iteratively training the S-DNN (604) and OD-DNN (608).

**13.** The method (700) of any of the preceding claim 8 to claim 12, further comprising: generating multiple versions of each image in the training data set for augmenting the training data set.

**14.** A computer program product comprising instructions which, when the program is executed by one or more computers, cause the one or more computers to carry out steps (702, 704, 706, 708, 710, 712) of the method (700) according to any of the preceding claim 8 to claim 13.

**15.** A system (800) comprising one or more computers (801) and one or more storage devices (802) storing instructions that when executed by the one or more computers, cause the one or more computers to perform the method (700) of any of claim 8 to claim 13.

**Patentansprüche**

**1.** Von einem oder mehreren Computern durchgeführtes Verfahren (100) zum Ausgeben einer Messung (610) einer Abmessung eines Körperteils (602) in einem Eingangsbild (601), wobei das Verfahren umfasst:

Empfangen (102) des unter Verwendung einer Bilderfassungseinheit (603) erfassten Eingangsbilds, wobei das Eingangsbild den Körperteil und ein Referenzobjekt (607) einer vordefinierten Abmessung umfasst;

Vorhersagen (104) einer Segmentierungsmaske (605) des Eingangsbilds durch ein tiefes neuronales Netzwerk zur Segmentierung, S-DNN (604), wobei die Segmentierungsmaske Bildpixel des Körperteils von Bildpixeln eines Hintergrundbereichs in dem Eingangsbild unterscheidet;

Erkennen des Referenzobjekts in dem Eingangsbild und Verarbeiten des erkannten Referenzobjekts zum Vorhersagen (106) einer Pixelabmessung (609) des Referenzobjekts durch ein tiefes neuronales Netzwerk zur Objekterkennung, OD-DNN (608);

Erhalten (108) von Pixelkoordinaten einer Vielzahl von Schlüsselpunkten (A, H, J, K, Q, T) des Körperteils unter Verwendung der Segmentierungsmaske;

Modifizieren (110) der erhaltenen Pixelkoordinaten auf der Grundlage der vorhergesagten Pixelabmessung des Referenzobjekts; und Messen der Abmessung des Körperteils auf der Grundlage der geänderten Pixelkoordinaten und Ausgeben (112) der gemessenen Abmessung des Körperteils.

**2.** Verfahren (100) nach Anspruch 1, wobei das S-DNN (604) ein semantisches S-DNN ist, sodass die Bildpixel des Körperteils (602) in der Segmentierungsmaske (605) sich auf eine erste Klasse beziehen und die Bildpixel des Hintergrundbereichs in der Segmentierungsmaske sich auf eine zweite Klasse beziehen.

**3.** Verfahren (100) nach Anspruch 1 oder Anspruch 2, wobei das OD-DNN (608) dazu konfiguriert ist, eine Messung pro Pixel, MPP, welche einer Abmessung der im Eingangsbild (601) gezeigten, durch ein einzelnes Pixel dargestellten Umgebung entspricht, auf der Grundlage der vordefinierten Abmessung und der vorhergesagten Pixelabmessung (609) des Referenzobjekts (607) zu berechnen.

**4.** Verfahren (100) nach einem der vorstehenden Ansprüche, weiter umfassend:
Verwenden von zumindest einem von dem Eingangsbild (601), der Segmentierungsmaske (605), der vorhergesagten Pixelabmessung (609) des Referenzobjekts (607) und der Ausgabemessung (610) der Abmessung des Körperteils (602) für eine zukünftige Vorhersage der Segmentierungsmaske und/oder der Pixelabmessung des Referenzobjekts.

**5.** Verfahren (100) nach einem der vorstehenden Ansprüche, wobei Erhalten (108) von Pixelkoordinaten einer Vielzahl von Schlüsselpunkten (A, H, J, K, Q, T) des Körperteils (602) unter Verwendung der Segmentierungsmaske (605) umfasst:

Definieren einer Vielzahl von Koordinatenachsen (x, y) auf der Segmentierungsmaske; Identifizieren von Bildpixeln, die lokale Maxima und/oder lokale Minima-Koordinaten entlang zumindest einer gewählten Koordinatenachse aus der Vielzahl von Koordinatenachsen aufweisen, von spezifischen Bereichen des Körperteils auf der Segmentierungsmaske und Erhalten der Pixelkoordinaten der Vielzahl von Schlüsselpunkten des Körperteils auf der Grundlage der identifizierten Pixel der spezifischen Bereiche.

**6.** Computerprogrammprodukt, umfassend Anweisungen, welche, wenn das Programm von einem oder mehreren Computern ausgeführt wird, den einen oder die mehreren Computer veranlassen, Schritte (102, 104, 106, 108, 110, 112) des Verfahrens (100) nach einem der vorstehenden Ansprüche auszuführen.

7. System (600), umfassend einen oder mehrere Computer und eine oder mehrere Speichervorrichtungen, welche Anweisungen speichern, welche, wenn sie von dem einen oder den mehreren Computern ausgeführt werden, den einen oder die mehreren Computer veranlassen, das Verfahren (100) nach einem von Anspruch 1 bis Anspruch 5 durchzuführen.

8. Computerimplementiertes Verfahren (700) zum Trainieren einer Vielzahl von neuronalen Netzwerken (604, 608), welche zum Steuern einer elektronischen Vorrichtung (600) verwendet werden, um eine Messung (610) einer Abmessung eines Körperteils (602) in einem Eingangsbild (601) auszugeben, wobei das Eingangsbild von einer Bilderfassungseinheit (603) der elektronischen Vorrichtung erfasst wird, wobei die Vielzahl von neuronalen Netzwerken ein tiefes neuronales Netzwerk zur Objekterkennung, OD-DNN (608) und ein tiefes neuronales Netzwerk zur Segmentierung, S-DNN (604), umfasst, wobei das Verfahren die folgenden Schritte umfasst:

    Initialisieren (702) der neuronalen Netzwerkparameterwerte des S-DNN und des OD-DNN;
    Erhalten (704) eines Trainingsdatensatzes, welcher eine Vielzahl von Bildern umfasst, wobei jedes Bild den Körperteil und ein Referenzobjekt (607) mit einer vordefinierten Abmessung beinhaltet;
    Kommentieren (706) des Referenzobjekts und des Körperteils in den Bildern des Trainingsdatensatzes, um einen kommentierten Trainingsdatensatz zu generieren;
    Aufteilen (708) eines Teils des kommentierten Trainingsdatensatzes in einen Validierungsdatensatz;
    Eingeben (710) des kommentierten Trainingsdatensatzes in das OD-DNN, sodass das OD-DNN lernt, das Referenzobjekt in dem Eingangsbild zu erkennen und das erkannte Referenzobjekt zu verarbeiten, um eine Pixelabmessung (609) des Referenzobjekts vorherzusagen;
    Eingeben (710) des kommentierten Trainingsdatensatzes in das S-DNN, sodass das S-DNN lernt, eine Segmentierungsmaske (605) des Eingangsbilds vorherzusagen, wobei die Segmentierungsmaske Bildpixel des Körperteils von Bildpixeln eines Hintergrundbereichs in dem Eingangsbild unterscheidet;
    Validieren (712) einer von dem OD-DNN erlernten Pixelabmessung des Referenzobjekts und einer von dem S-DNN erlernten Segmentierungsmaske unter Verwendung des Validierungsdatensatzes, und
    Aktualisieren (714) der neuronalen Netzwerkparameterwerte des S-DNN und des OD-DNN auf der Grundlage der Validierung.

9. Verfahren (700) nach Anspruch 8, wobei das OD-DNN (608) und das S-DNN (604) vorab trainiert werden, um Objekte in einem beliebigen Bild zu erkennen und ein beliebiges Bild semantisch in jeweils zumindest eine Segmentmaske einer spezifischen Bildklasse zu segmentieren.

10. Verfahren (700) nach Anspruch 8 oder Anspruch 9, wobei Kommentieren (706) des Referenzobjekts (607) und des Körperteils (602) in dem erhaltenen Trainingsdatensatz jeweils umfasst:

    Kommentieren des in jedem Bild des Trainingsdatensatzes umfassten Referenzobjekts unter Verwendung eines Begrenzungsrahmens;
    Kommentieren von Bildpixeln des Körperteils in jedem Bild des Trainingsdatensatzes, wobei sich der Körperteil auf eine erste Klasse bezieht.

11. Verfahren (700) nach einem der vorstehenden Anspruch 8 bis Anspruch 10, wobei das OD-DNN (608) weiter gelehrt wird, eine Messung pro Pixel (MPP), welche einer Abmessung der im Eingangsbild (601) gezeigten, durch ein einzelnes Pixel dargestellten Umgebung entspricht, auf der Grundlage der vordefinierten Abmessung und der vorhergesagten Pixelabmessung (609) des Referenzobjekts (607) zu berechnen.

12. Verfahren (700) nach einem der vorstehenden Anspruch 8 bis Anspruch 11, welches weiter periodisches Erhalten des Trainingsdatensatzes umfasst, welcher die Vielzahl von Bildern zum iterativen Trainieren des S-DNN (604) und des OD-DNN (608) umfasst.

13. Verfahren (700) nach einem der vorstehenden Anspruch 8 bis Anspruch 12, weiter umfassend: Generieren mehrerer Versionen jedes Bildes in dem Trainingsdatensatz zum Erweitern des Trainingsdatensatzes.

14. Computerprogrammprodukt, umfassend Anweisungen, welche, wenn das Programm von einem oder mehreren Computer ausgeführt wird, den einen oder die mehreren Computer veranlassen, Schritte (702, 704, 706, 708, 710, 712) des Verfahrens (700) nach einem der vorstehenden Anspruch 8 bis Anspruch 13 auszuführen.

15. System (800), umfassend einen oder mehrere Computer (801) und eine oder mehrere Speichervorrichtungen (802), welche Anweisungen speichern, welche, wenn sie von dem einen oder den mehreren Computern ausgeführt werden, den einen oder die mehreren Computer veranlassen, das Verfahren (700) nach einem von Anspruch 8 bis Anspruch 13 durchzuführen.

**Revendications**

1. Procédé (100) réalisé par un ou plusieurs ordinateurs pour délivrer une mesure (610) d'une dimension d'une partie de corps (602) dans une image d'entrée (601), le procédé comprenant :

   la réception (102) de l'image d'entrée capturée à l'aide d'une unité de capture d'image (603), l'image d'entrée comprenant la partie de corps et un objet de référence (607) d'une dimension prédéfinie ;
   la prédiction (104), par un réseau neuronal profond de segmentation, S-DNN (604), d'un masque de segmentation (605) de l'image d'entrée, le masque de segmentation différenciant des pixels d'image de la partie de corps de pixels d'image d'une région d'arrière-plan dans l'image d'entrée ;
   la détection de l'objet de référence dans l'image d'entrée et le traitement de l'objet de référence détecté pour prédire (106) une dimension de pixel (609) de l'objet de référence par un réseau neuronal profond de détection d'objet, OD-DNN (608) ;
   l'obtention (108) de coordonnées de pixel d'une pluralité de points clés (A, H, J, K, Q, T) de la partie de corps à l'aide du masque de segmentation ;
   la modification (110) des coordonnées de pixel obtenues sur la base de la dimension de pixel prédite de l'objet de référence ; et
   la mesure de la dimension de la partie de corps sur la base des coordonnées de pixel modifiées et la sortie (112) de la dimension mesurée de la partie de corps.

2. Procédé (100) selon la revendication 1, dans lequel le S-DNN (604) est un S-DNN sémantique de telle sorte que les pixels d'image de la partie de corps (602) dans le masque de segmentation (605) se rapportent à une première classe et les pixels d'image de la région d'arrière-plan dans le masque de segmentation se rapportent à une seconde classe.

3. Procédé (100) selon la revendication 1 ou la revendication 2,
   dans lequel l'OD-DNN (608) est configuré pour calculer une mesure par pixel, MPP, correspondant à une dimension de l'environnement montré dans l'image d'entrée (601) représentée par un seul pixel, sur la base de la dimension prédéfinie et de la dimension de pixel (609) prédite de l'objet de référence (607).

4. Procédé (100) selon l'une quelconque des revendications précédentes, comprenant en outre :
   l'utilisation d'au moins l'un de l'image d'entrée (601), du masque de segmentation (605), de la dimension de pixel prédite (609) de l'objet de référence (607) et de la mesure de sortie (610) de la dimension de la partie de corps (602) pour une prédiction future du masque de segmentation et/ou de la dimension de pixel de l'objet de référence.

5. Procédé (100) selon l'une quelconque des revendications précédentes, dans lequel l'obtention (108) de coordonnées de pixel d'une pluralité de points clés (A, **H**, J, K, Q, T) de la partie de corps (602) à l'aide du masque de segmentation (605) comprend :

   la définition d'une pluralité d'axes de coordonnées (x, y) sur le masque de segmentation ;
   l'identification de pixels d'image présentant des coordonnées de maxima locaux et/ou de minima locaux le long d'au moins un axe de coordonnées choisi parmi la pluralité d'axes de coordonnées, de régions spécifiques de la partie de corps sur le masque de segmentation et
   sur la base des pixels identifiés des régions spécifiques, l'obtention des coordonnées de pixel de la pluralité de points clés de la partie de corps.

6. Produit de programme informatique comprenant des instructions qui, lorsque le programme est exécuté par un ou plusieurs ordinateurs, amènent les un ou plusieurs ordinateurs à effectuer des étapes (102, 104, 106, 108, 110, 112) du procédé (100) selon l'une quelconque des revendications précédentes.

7. Système (600) comprenant un ou plusieurs ordinateurs et un ou plusieurs dispositifs de stockage stockant des instructions qui, lorsqu'elles sont exécutées par les un ou plusieurs ordinateurs, amènent les un ou plusieurs ordinateurs à effectuer le procédé (100) selon l'une quelconque de la revendication 1 à la revendication 5.

8. Procédé mis en œuvre par ordinateur (700) d'entraînement d'une pluralité de réseaux neuronaux (604, 608) utilisés pour commander un dispositif électronique (600) pour délivrer une mesure (610) d'une dimension d'une partie de corps (602) dans une image d'entrée (601), l'image d'entrée étant capturée par une unité de capture d'image (603) du dispositif électronique, la pluralité de réseaux neuronaux comprenant un réseau neuronal profond de détection d'objet, OD-DNN (608) et un réseau neuronal profond de segmentation, S-DNN (604), le procédé comprenant les étapes suivantes :

> l'initialisation (702) de valeurs de paramètre de réseau neuronal du S-DNN et de l'OD-DNN ;
> l'obtention (704) d'un jeu de données d'entraînement comprenant une pluralité d'images, chaque image incluant la partie de corps et un objet de référence (607) d'une dimension prédéfinie ;
> l'annotation (706) de l'objet de référence et de la partie de corps dans les images du jeu de données d'entraînement pour générer un jeu de données d'entraînement annoté ;
> la division (708) d'une partie du jeu de données d'entraînement annoté en un jeu de données de validation ;
> l'entrée (710) du jeu de données d'entraînement annoté dans l'OD-DNN de telle sorte que l'OD-DNN apprend à détecter l'objet de référence dans l'image d'entrée et à traiter l'objet de référence détecté pour prédire une dimension de pixel (609) de l'objet de référence ;
> l'entrée (710) du jeu de données d'entraînement annoté dans le S-DNN de telle sorte que le S-DNN apprend à prédire un masque de segmentation (605) de l'image d'entrée, dans lequel le masque de segmentation différencie des pixels d'image de la partie de corps de pixels d'image d'une région d'arrière-plan dans l'image d'entrée ;
> la validation (712), à l'aide du jeu de données de validation, d'une dimension de pixel de l'objet de référence appris par l'OD-DNN et d'un masque de segmentation appris par le S-DNN, et
> la mise à jour (714) des valeurs de paramètre de réseau neuronal du S-DNN et de l'OD-DNN sur la base de la validation.

9. Procédé (700) selon la revendication 8, dans lequel l'OD-DNN (608) et le S-DNN (604) sont préalablement entraînés pour détecter des objets dans une image arbitraire et segmenter sémantiquement une image arbitraire en au moins un masque de segment d'une classe d'image spécifique, respectivement.

10. Procédé (700) selon la revendication 8 ou la revendication 9, dans lequel l'annotation (706) de l'objet de référence (607) et de la partie de corps (602) dans le jeu de données d'entraînement obtenu comprend respectivement :

> l'annotation de l'objet de référence compris dans chaque image du jeu de données d'entraînement à l'aide d'un cadre de délimitation ;
> l'annotation de pixels d'image de la partie de corps dans chaque image du jeu de données d'entraînement, dans lequel la partie de corps se rapporte à une première classe.

11. Procédé (700) selon l'une quelconque de la précédente revendication 8 à la précédente revendication 10, dans lequel l'OD-DNN (608) apprend en outre à calculer une mesure par pixel (MPP) correspondant à une dimension de l'environnement montré dans l'image d'entrée (601) représentée par un seul pixel, sur la base de la dimension prédéfinie et de la dimension de pixel prédite (609) de l'objet de référence (607).

12. Procédé (700) selon l'une quelconque de la précédente revendication 8 à la précédente revendication 11, comprenant en outre l'obtention périodique du jeu de données d'entraînement comprenant la pluralité d'images pour entraîner de manière itérative le S-DNN (604) et l'OD-DNN (608).

13. Procédé (700) selon l'une quelconque de la précédente revendication 8 à la précédente revendication 12, comprenant en outre : la génération de multiples versions de chaque image dans le jeu de données d'entraînement pour augmenter le jeu de données d'entraînement.

14. Produit de programme informatique comprenant des instructions qui, lorsque le programme est exécuté par un ou plusieurs ordinateurs, amènent les un ou plusieurs ordinateurs à effectuer des étapes (702, 704, 706, 708, 710, 712) du procédé (700) selon l'une quelconque de la précédente revendication 8 à la précédente revendication 13.

15. Système (800) comprenant un ou plusieurs ordinateurs (801) et un ou plusieurs dispositifs de stockage (802) stockant des instructions qui, lorsqu'elles sont exécutées par les un ou plusieurs ordinateurs, amènent les un ou plusieurs ordinateurs à effectuer le procédé (700) selon l'une quelconque de la revendications 8 à la revendication 13.

100

RECEIVE THE INPUT IMAGE COMPRISING A BODY PART AND A REFERENCE OBJECT OF A PREDEFINED DIMENSION 102

↓

PREDICT A SEGMENTATION MASK OF THE INPUT IMAGE 104

↓

PREDICT A PIXEL DIMENSION OF THE REFERENCE OBJECT 106

↓

OBTAIN PIXEL COORDINATES OF A PLURALITY OF KEYPOINTS OF THE BODY PART USING THE SEGMENTATION MASK 108

↓

MODIFY THE OBTAINED PIXEL COORDINATES BASED ON THE PREDICTED PIXEL DIMENSION OF THE REFERENCE OBJECT 110

↓

OUTPUT MEASUREMENT OF THE DIMENSION OF THE BODY PART BASED ON THE MODIFIED PIXEL COORDINATES 112

**FIG. 1**

**FIG. 2**

**FIG. 3**

inches/pixel

**FIG. 4**

FIG. 5A

FIG. 5B

FIG. 6

EP 4 436 473 B1

700

INITIALIZE NEURAL NETWORK PARAMETER VALUES OF THE NEURAL NETWORKS
702

↓

OBTAIN TRAINING DATA SET COMPRISING A PLURALITY OF IMAGES 704

↓

ANNOTATE THE TRAINING DATA SET  706

↓

DIVIDE A PART OF THE ANNOTATED TRAINING DATA SET INTO A VALIDATION
DATA SET 708

↓

INPUT THE ANNOTATED TRAINING DATA SET TO THE NEURAL NETWORKS 710

↓

VALIDATE, USING THE VALIDATION DATA SET, OUTPUT OF NEURAL NETWORKS
712

↓

UPDATE THE NEURAL NETWORK PARAMETER VALUES BASED ON THE
VALIDATION 714

**FIG. 7**

Training system 800

1000

600

1002

Training engine 801 ⟷ Neural network 803

Memory 802

**FIG. 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Segmenting skin ulcers and measuring the wound area using deep convolutional networks. **CHINO DANIEL et al.** COMPUTER METHODS AND PROGRAMS IN BIOMEDICINE. ELSEVIER, 07 February 2020, vol. 191

- Automatic measurement of anthropometric dimensions using frontal and lateral silhouettes. **ASLAM MURTAZA et al.** IET COMPUTER VISION, THE INSTITUTION OF ENGINEERING AND TECHNOLOGY, MICHAEL FARADAY HOUSE, SIX HILLS WAY, STEVENAGE, HERTS. September 2017, vol. 11, 434-447